# EUROPÄISCHE PATENTANMELDUNG

(11) **EP 1 110 500 A2**
(43) Veröffentlichungstag der Anmeldung: **27.06.2001**
(21) Anmeldenummer: 00126850.7
(22) Anmeldetag: 07.12.2000
(51) Int. Cl.: A61B 5/00, A61B 5/16

(54) **Verfahren und Vorrichtung zur Selbstkontrolle des Ist-Zustands eines an einer neurologischen Erkrankung leidenden Patienten**

(30) Priorität: 20.12.1999 DE 19961526
(71) Anmelder: SIEMENS AKTIENGESELLSCHAFT, 80333 München (DE)
(72) Erfinder: Abraham-Fuchs, Klaus, 91058 Erlangen (DE); Schmidt, Kai-Uwe, Dr., 91052 Erlangen (DE)

(57) **Zusammenfassung**

Verfahren zur Selbstkontrolle des Ist-Zustands eines an einer neurologischen Erkrankung leidenden, medikamentös therapierten Patienten, bei welchem unter Verwendung einer patientenseitig angeordneten Rechnereinrichtung (1) Informationen betreffend die motorischen und/oder verbalen und/oder kognitiven Fähigkeiten des Patienten interaktiv erfasst werden und basierend auf diesen Informationen mittels eines Expertensystems (10) wenigstens eine den Zustand beschreibende Maßzahl oder eine Aussage ermittelt und an den Patienten mittels eines rechnerseitigen Ausgabemediums (2) ausgegeben wird.

## Beschreibung

Die Erfindung betrifft ein Verfahren zur Selbstkontrolle des Ist-Zustands eines an einer neurologischen Erkrankung leidenden, medikamentös therapierten Patienten.

Patienten mit neurologischen oder psychiatrischen Erkrankungen werden häufig über einen langen Zeitraum mit Medikamenten (z.B. Psychopharmaka aus der Klasse der Neuroleptika) behandelt, die die geistigen Fähigkeiten wie Reaktionsfähigkeiten, Koordinationsfähigkeit, sprachliche Artikulationsfähigkeit, Erinnerungsvermögen, kognitive Fähigkeiten etc. negativ beeinflussen. Betroffene Krankheitsbilder sind z.B. Epilepsie, Depression, Schizophrenie, Schlafstörungen etc. Die ärztliche Kunst besteht bei einer solchen medikamentösen Therapie darin, die Dosierung und Dauer der Einnahme der Medikamente so zu wählen, dass einerseits die negativen Auswirkungen der Krankheit bestmöglich unterdrückt werden (z.B. epileptische Anfälle), andererseits die Lebensqualität des Patienten durch Einschränkung der oben genannten natürlichen Fähigkeiten des Patienten so wenig als möglich beeinträchtigt wird.

Diese Balance zwischen erwünschter Wirkung und negativer Begleiterscheinung des Medikaments wird als optimale Medikamenteneinstellung bezeichnet. Insbesondere wenn die Medikamente über einen langen Zeitraum (mehrere Wochen oder Monate) eingenommen werden, kann sich das Optimum der Dosierung ständig verändern, etwa durch Gewöhnung des Patienten an das Medikament oder durch Veränderung des Krankheitszustands. Daher besteht bei solchen Langzeittherapien der Bedarf, eine regelmäßige Kontrollmöglichkeit für die Optimierung der Medikamenteneinstellung zu haben. Wünschenswert ist es insbesondere, dass der Arzt eine solche Kontrolle der Wirkung der Medikamente nicht nur sporadisch bei Besuchen des Patienten in der Arztpraxis vornehmen kann, sondern vielmehr in kürzeren Abständen und vor allem unter Alltagsbedingungen zu Hause eine solche Kontrolle erfolgt.

Wenn die Nebenwirkungen des Medikaments mit Veränderungen von metabolischen Parametern, z.B. im Blut oder Urin korrelieren, kann eine solche Medikamenteneinstellung durch Kontrolle von Labormesswerten vorgenommen werden, was jedoch sehr aufwendig ist. Wenn jedoch höhere Funktionen des menschlichen Organismus, wie die genannten neurologischen Fähigkeiten, beeinflusst werden, so ist der Grad der Beeinflussung nicht mehr durch einfache Labormesswerte, elektrophysiologischer Messwerte etc. erfassbar. Vielmehr ist es notwendig, die beeinträchtigte neurologische Funktion des Patienten als solche zu beurteilen.

Aus der Zeitschrift "Forschung und Innovation", II/98, Verlag Siemens AG, Unternehmenskommunikation, Cooperate Publishing Erlangen, Bestell Nr. A 19100-L 516-U 982, Seite 18-22 ist ein System bekannt, das Anamnesedaten abfragen kann und eine Analyse des Gesundheitszustandes angeben kann. Dieses System ist ferner in der Lage, beispielsweise Kinder aufzufordern, einen Tennisball auf einem Bildschirm wegzuschlagen, um so die Eltern frühzeitig auf eventuelle neurologische Probleme aufmerksam zu machen. Auch besteht die Möglichkeit, mit dem System Seh- und Hörtests durchzuführen. Eine echte Selbstkontrolle für den Patienten, die es ihm ermöglicht, sich ein Bild über seinen Ist-Zustand zu verschaffen, ist hiermit jedoch nicht möglich.

Der Erfindung liegt damit das Problem zugrunde, ein Verfahren sowie ein System zur Durchführung des Verfahrens anzugeben, welches es dem Patienten auf einfache Weise ermöglicht, sich selbst rasch ein Bild über seinen Ist-Zustand zu verschaffen.

Zur Lösung dieses Problems ist ein Verfahren zur Selbstkontrolle des Ist-Zustands eines an einer neurologischen Erkrankung leidenden, medikamentös therapierten Patienten vorgesehen, bei welchem unter Verwendung einer patientenseitig angeordneten Rechnereinrichtung Informationen betreffend die motorischen, verbalen und/oder kognitiven Fähigkeiten des Patienten interaktiv erfasst werden und basierend auf diesen Informationen mittels eines Expertensystems wenigstens eine den Zustand beschreibende Maßzahl oder eine Aussage ermittelt und an den Patienten mittels eines rechnerseitigen Ausgabemediums ausgegeben wird.

Beim erfindungsgemäßen Verfahren muss der Patient interaktiv mit der Rechnereinrichtung kommunizieren. Rechnerseitig werden vorteilhaft spezifische Informationen, die quasi ein Maß für die momentanen motorischen, verbalen und/oder kognitiven Fähigkeiten des Patienten darstellen oder liefern, erfasst und in einem Expertensystem, welches beispielsweise als neuronales Netz ausgebildet sein kann, verarbeitet, um eine Quantifizierung des Zustands des Patienten hinsichtlich seiner motorischen/verbalen/kognitiven Fähigkeiten vorzunehmen. Das Ergebnis der Verarbeitung im Expertensystem kann vorteilhaft ein den Zustand beschreibende Maßzahl (z.B. eine Zahl aus dem Bereich 0 - 100, wobei "0" für einen sehr schlechten und "100" für einen sehr guten Zustand steht) oder eine Aussage sein, die dem Patienten eine Information über sein Ist-Zustand liefert. Anhand der ermittelten Maßzahl oder der Aussage ist es dem Patienten möglich, seinen Ist-Zustand zu erkennen, gegebenenfalls unter Vergleich mit einer während der letzten Kontrolle ermittelten Maßzahl oder Aussage, so dass er entsprechend reagieren kann. Die Reaktion kann unterschiedlicher Art sein. Entweder bleibt die Medikamentendosierung wie sie war, was dann der Fall ist, wenn sich der Zustand nicht verschlechtert hat oder sogar verbessert hat. Ferner kann als Reaktion eine Erhöhung der Medikamentendosierung vom Patienten vorgenommen werden, wenn sich beispielsweise sein Zustand verschlechtert hat oder aber die letzte Erhöhung ebenfalls nicht angeschlagen hat, gleichwie auch eine Dosiserniedrigung erfolgen kann, wenn sich im Vergleich zur letzten oder den letzten Zustandsbeurteilungen eine deutliche Besserung ergeben hat.

Der Patient ist mit dem erfindungsgemäßen Verfahren vorteilhaft in der Lage, auf einfache und schnelle Weise Informationen über seinen Ist-Zustand zuhause zu erhalten, so dass er zum einen in beliebigen kurzen Abständen selbstentsprechende Kenntnis über seinen Zustand oder sein Befinden erhalten kann, was ansonsten lediglich in relativ langen Zeitabständen durch Arztbesuche der Fall ist, zum anderen können diese Arztbesuche unterbleiben, wenn das Untersuchungsergebnis eine Zustandsverbesserung oder Stabilität oder dergleichen anzeigt.

Gemäß einer zweckmäßigen Erfindungsausgestaltung kann vorgesehen sein, dass der Patient zur Erfassung der die Motorik betreffenden Informationen an der Rechnereinrichtung softwaregesteuerte Motoriktestübungen durchführt, wobei die Motoriktestübungen zur Erfassung der negativen und/oder der positiven Auswirkungen einer Medikamentengabe auf den Patientenzustand dienen, und anhand welcher ein die negativen und/oder positiven Auswirkungen quantifizierender Informationswert ermittelt wird, der vom Expertensystem weiterverarbeitet wird.

Der Patient wird gefordert, entsprechende, durch geeignete an der Rechnereinrichtung hinterlegte Software gesteuerte Motoriktestübungen durchzuführen, die ihm beispielsweise nach Art eines Computerspiels an einem Monitor angezeigt werden. Dabei kann es sich beispielsweise um Reaktionsübungen handeln, oder um Positionierungsübungen, im Rahmen welcher der Patient beispielsweise mit einem am Monitor gezeigten Curser bestimmte vorgegebene Positionen mit einer Bedienmaus anfahren muss oder dergleichen. Einsetzbar und zur Informationserfassung dienlich sind sämtliche Übungen, die in irgendeiner Form die Motorik des Patienten fordern. Dabei können die Testübungen derart spezifisch aufgebaut sein, dass sie zu konkreten Ermittlungen negativer oder positiver Auswirkungen dienen. Der Informationswert kann seitens des die Testübungen steuernden Softwareprogramms selbst ermittelt werden, alternativ kann hierzu auch eine separate geeignete Software hinterlegt sein. Natürlich besteht auch die Möglichkeit, dass die Informationsrohdaten direkt dem Expertensystem gegeben werden, welches unter Auslassung des genannten quantifizierenden Informationswerts die direkte Verarbeitung vornimmt.

Zusätzlich oder alternativ kann vorgesehen sein, dass zur Erfassung der die verbalen Fähigkeiten des Patienten betreffenden Informationen ein rechnerseitig hinterlegtes Sprachbeurteilungssystem verwendet wird, das Spracherkennungsalgorithmen und/oder eine phonetische Datenbank enthält, mittels welchem ein die negativen und/oder positiven Auswirkungen einer Medikamentengabe quantifizierender Informationswert der Artikulationsfähigkeit ermittelt wird, der vom Expertensystem weiterverarbeitbar ist. Mittels des Sprachbeurteilungssystems können dem Patienten vorgegebene Sätze akustisch vorgespielt werden, die er nachzusprechen hat. Dies wird mittels einer entsprechenden Sprachaufzeichnungseinrichtung aufgenommen und mittels der Spracherkennungsalgorithmen bzw. der phonetischen Datenbank ausgewertet und ein quantifizierender Informationswert ermittelt. Im Rahmen dieser Ermittelung kommt es beispielsweise auf die Geschwindigkeit der vom Patienten gesprochenen Wortfolge an, auf die Genauigkeit der Aussprache und die Betonung etc. Auch hier besteht selbstverständlich die Möglichkeit, dass das Expertensystem direkt die aufgenommenen Informationen nach einer ersten Beurteilung durch das Sprachbeurteilungssystem ohne Ermittlung eines Informationswerts weiterverarbeitet werden.

Um schließlich die kognitiven Fähigkeiten beurteilen zu können, können in Weiterbildung des Erfindungsgedankens softwaregesteuert im Frage-Antwort-Verfahren vorgegebene Fragen optisch oder akustisch an den Patienten ausgegeben werden, welche der Patient verbal oder manuell rechnerseitig beantworten muss, wobei basierend auf die Antworten ein die negativen und/oder positiven Auswirkungen quantifizierender Informationswert der kognitiven Fähigkeiten ermittelt wird, der vom Expertensystem weiterverarbeitet wird. Bei den an den Patienten gestellten Fragen kann es sich um beliebige Wissensfragen handeln, die einerseits abhängig vom jeweiligen Bildungsgrad des Patienten, andererseits natürlich auch abhängig vom Krankheitsbild des Patienten im Schwierigkeitsgrad bemessen sein können.

In Weiterbildung des Erfindungsgedankens kann vorgesehen sein, dass zusätzlich zu den genannten Informationen unter Verwendung eines Erfassungssystems rechnerseitig einzugebende oder verbal erfassbare Informationen betreffend das subjektive Befinden des Patienten erfasst werden, die seitens des Expertensystems weiterverarbeitet werden. Bei diesen Daten handelt es sich um allgemeine Anamnesedaten. Um diese abzufragen sind in Form eines geeigneten Softwareprogramms beispielsweise vorgegebene Fragebögen hinterlegt, die dem Patienten am Monitor visualisiert werden, und die er zu beantworten bzw. auszufüllen hat, auch wissensbasierte Dialogsysteme etc. sind denkbar.

Insgesamt ist festzuhalten, dass die Informationserfassung stets unter Verwendung geeigneter Softwareprogramme erfolgt. Das Expertensystem ist in der Lage, aus der Summe der erfassten Informationen bzw. Übungsergebnisse sowie der erfassten verbalen Informationen eine entsprechende Maßzahl oder Aussage abzuleiten, die den Patienten über seinen Ist-Zustand informiert. Die abgeleiteten Maßzahlen oder Aussagen können zweckmäßig auch gespeichert und im Zeitverlauf dargestellt werden, so dass auch langfristige Trendaussagen über die Entwicklung des Patientenzustands ermöglicht werden.

Als zweckmäßig hat es sich erwiesen, wenn das Expertensystem anhand der gegebenen Maßzahl oder Aussage oder der Informationen bzw. der ermittelten Informationswerte eine Bewertung betreffend die Medikamentendosierung vornimmt und ausgibt. Gemäß dieser Erfindungsausgestaltung ist das Expertensystem derart ausgeprägt, dass es anhand der gegebenen Daten einen Vorschlag betreffend die Medikamentendosierung dahingehend macht, ob diese richtig, zu niedrig oder zu hoch ist, so dass der Patient hieraus einen ersten Anhaltspunkt gewinnt, wie er sich zukünftig verhalten sollte. Die Ermittlung der Bewertung kann anhand der einen, im Rahmen der vorher erfolgten Kontrollsitzung ermittelten Maßzahl oder Aussage erfolgen, sofern diese in ihrem Informationsgehalt hinreichend ist, eine fundierte und sinnvolle Bewertung vorzunehmen. Zweckmäßig ist es insbesondere, wenn zur Ermittlung der Bewertung bereits früher aufgenommene Maßzahlen/Aussagen mit beachtet werden und der zeitliche Verlauf bzw. die Änderung seitens des Expertensystems analysiert und der zeitliche Trend erfasst wird, und die Bewertung gestützt auf das Analyseergebnis erfolgt. Denn auch der zeitliche Verlauf kann wichtige Informationen offenbaren, die in eine sinnvolle Bewertung mit eingehen sollten. Auch ist zu Kontrollzwecken eine Darstellung des zeitlichen Verlaufs der Maßzahlen am Ausgabemedium zweckmäßig, da der Patient schnell den Therapieerfolg erfassen kann.

Neben der Tatsache, dass sich der Patient auf einfache und schnelle Weise einen hinreichend gesicherten Überblick über seinen Ist-Zustand verschaffen kann, wäre es ebenfalls vorteilhaft, wenn der behandelnde Arzt gleichermaßen diese Information erhalten würde. Um dem nachzukommen kann erfindungsgemäß vorgesehen sein, dass die Maßzahl oder die Aussage, gegebenenfalls auch die Bewertung betreffend die Medikamentendosierung und gegebenenfalls die Informationen selbst über eine Kommunikationsverbindung an den Arzt übertragen und dort an einem Anzeigemedium, z.B. einem Monitor angezeigt werden. Dieser hat damit sowohl die Möglichkeit einer telemedizinischen Verlaufskontrolle und kann den Patienten beim nächsten Besuch besser beurteilen und beraten. Für den Fall, dass der Arzt anhand der gegebenen Maßzahlen/Aussagen etc. einen handlungsbedürftigen Befund erstellt, kann er ebenfalls sofort einschreiten. Zusätzlich kann erfindungsgemäß die Möglichkeit bestehen, dass der Arzt über die Kommunikationsverbindung in Ansehung die übertragenen Maßzahlen oder Aussagen oder Informationen eine Beurteilung des Patientenzustands vornimmt und eine Therapieanweisung an den Patienten übermittelt, die dem Patienten am Ausgabemedium angezeigt werden. Auf diese Weise ist es möglich, mit dem Patienten sofort in Kontakt treten zu können und gegebenenfalls wichtige Therapieanweisungen und Maßregeln zu übermitteln. Zweckmäßig ist es, wenn bei Erfassung der patientenseitigen Informationen bzw. unmittelbar nach Ermittlung der Maßzahlen oder Aussagen automatisch eine Datenübertragung an den Arzt erfolgt, so dass sichergestellt ist, dass dieser auch tatsächlich die Informationen erhält und für die Übertragung nicht der Patient verantwortlich ist, der dieser Verantwortung möglicherweise nicht immer nachkommen kann.

Neben dem Verfahren betrifft die Erfindung ferner ein System zur Durchführung des Verfahrens, umfassend eine patientenseitig angeordnete Rechnereinrichtung, in welcher ein oder mehrere Softwareprogramme abgelegt sind, mittels denen dem Patienten an einem oder mehreren Ausgabemedien interaktiv Testübungen betreffend die motorischen, verbalen und/oder kognitiven Fähigkeiten des Patienten optisch und/oder akustisch ausgebbar sind, wobei die Rechnereinrichtung zur Erfassung der Antwortinformationen des Patienten ausgebildet ist, und wobei rechnerseitig ein Expertensystem vorgesehen ist, das zur Ermittlung wenigstens einer an einem Ausgabemedium ausgebbaren, den Zustand des Patienten beschreibenden Maßzahl oder einer Aussage basierend auf den Antwortinformationen ausgebildet ist. Rechnerseitig sind also geeignete Softwareprogramme hinterlegt, die zur Ausgabe entsprechender Testübungen betreffend die motorischen, verbalen und kognitiven Fähigkeiten des Patienten dienen, wobei die Ausgabe an einem Monitor oder auch akustisch erfolgen kann. Ferner ist die Rechnereinrichtung zur Aufnahme entsprechender Antwortreaktionen des Patienten ausgebildet, d.h., softwareseitig werden die motorischen Bewegungsmuster als Reaktion auf die ausgegebene Motoriktestübung erfasst, alternativ dazu kann mit einem Sprachbeurteilungssystem die verbalen Fähigkeiten beurteilt werden und dergleichen. Das hinterlegte Expertensystem dient schließlich zur Verarbeitung der Informationen, um basierend darauf eine Maßzahl bzw. Aussage zu ermitteln, die den Patientenzustand quantifizierend beschreibt. Die hinterlegten Softwareprogramme sind in ihren Eigenschaften in den abhängigen Unteransprüchen näher beschrieben. Auch kann ein rechnerseitiges Erfassungssystem für im Frage-Antwort-Verfahren zu erfassende Informationen betreffend das subjektive Befinden des Patienten aufgenommen werden, wozu ein entsprechendes Erfassungssystem vorgesehen ist. Auch diese Daten gehen in das Expertensystem ein.

Das Expertensystem selbst ist einerseits zur Ermittlung der beschriebenen Maßzahl oder Aussage ausgebildet, zusätzlich kann es auch zur Ermittlung einer Bewertung betreffende Medikamentendosierung anhand der erfassten Antwortinformationen bzw. der ermittelten Informationswerte bzw. der ermittelten Maßzahl oder Aussage ausgebildet sein. Hierzu ist das Expertensystem zweckmäßigerweise zur Analyse des zeitlichen Verlaufs bzw. der Änderungen der Maßzahlen bzw. Aussage, die hierzu in einem rechnerseitigen Speichermittel ablegbar sind, ausgebildet, wobei das Expertensystem die Bewertung unter Berücksichtigung des Analyseergebnisses erstellt. Zweckmäßigerweise können gegebenenfalls auch die Informationswerte und deren Bewertungsergebnisse abgelegt werden, um auch davon Langzeitverlaufskontrollen zu ermöglichen. Generell sind alle genannten "Parameter", sofern sie gespeichert werden, in Form von zeitbezogenen Verlaufsdiagrammen am Ausgabemedium ausgebbar.

Schließlich können auch Übertragungsmittel zum Übertragen der Maßzahl oder der Aussage, gegebenenfalls auch der Bewertung betreffend die Medikamentendosierung und gegebenenfalls der Information selbst über eine Kommunikationsverbindung an eine externe Rechnereinrichtung vorgesehen sein, wobei die Rechnereinrichtung zur automatischen Übertragung an eine vorgegebene externe Rechnereinrichtung ausgebildet sein kann.

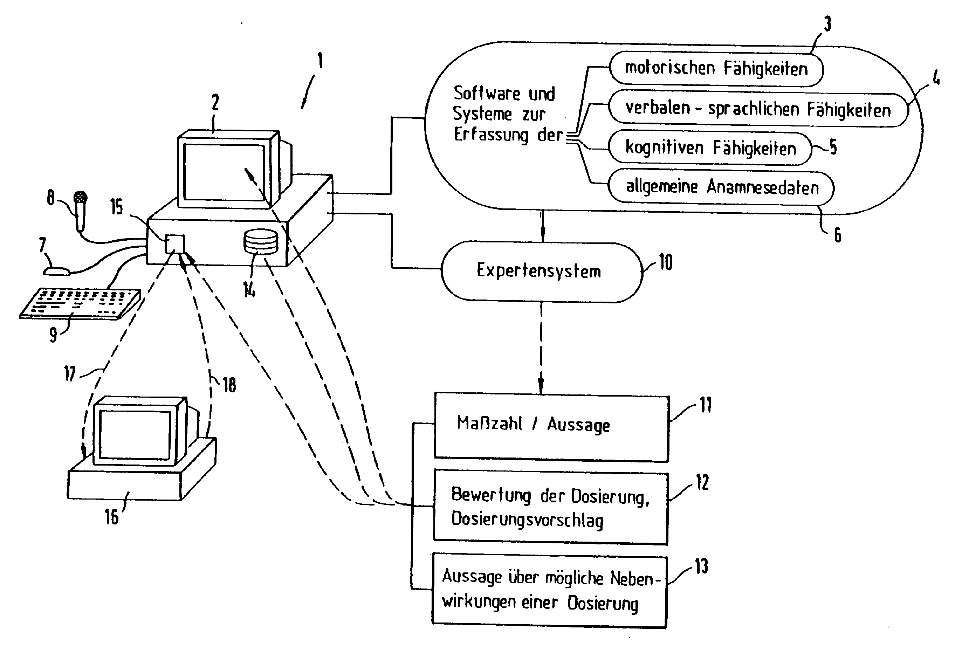

Weitere Vorteile, Merkmale und Einzelheiten der Erfindung ergeben sich aus dem im folgenden beschriebenen Ausführungsbeispiel sowie anhand der Zeichnung.

Diese zeigt eine Prinzipskizze zur Darstellung des erfindungsgemäßen Systems sowie zur Beschreibung des erfindungsgemäßen Verfahrens. Gezeigt ist eine Rechnereinrichtung 1 mit einem Ausgabemedium 2 in Form eines Monitors. Seitens der Rechnereinrichtung sind verschiedene Softwareprogramme und System zur Erfassung unterschiedlicher Informationen abgelegt, die eine Bewertung des Ist-Zustands eines neurologisch erkrankten Patienten, beispielsweise eines Epileptikers ermöglichen. Unter anderem ist geeignete Software und ein geeignetes System zur Erfassung motorischer Fähigkeiten 3, zur Erfassung verbaler-sprachlicher Fähigkeiten 4 zur Erfassung kognitiver Fähigkeiten 5 sowie zur Erfassung allgemeiner Anamnesedaten, die das subjektive Befinden des Patienten wiedergeben 6 in einem geeigneten Speichermittel abgelegt. Dem Patienten werden beispielsweise am Monitor 2 zur Erfassung der motorischen Fähigkeiten computerspielartige Aufgaben gestellt, die er bearbeiten muss. Beispielsweise kann es sich dabei um Reaktionsübungen und dergleichen handeln. Um die Aufgaben oder Übungen durchzuführen ist beispielsweise eine Bedienmaus 7 vorgesehen, mittels welcher ein Cursor oder dergleichen über den Monitor bewegt werden kann, und mit welchem beispielsweise einer vorgegebenen Linie, einem sich bewegenden Punkt und dergleichen gefolgt werden muss.

Ferner ist wie beschrieben entsprechende Software und ein System zur Erfassung der verbalen-sprachlichen Fähigkeiten hinterlegt. Bei dem System handelt es sich um ein Sprachbeurteilungssystem, welches Spracherkennungsalgorithmen und eine geeignete phonetische Datenbank erhält. Mit dem System sind vorprogrammierte Sprechbeispiele über einen nicht näher gezeigten Lautsprecher ausgebbar. Der Patient muss die vorgegebenen Sprechbeispiele nachsprechen, wozu beispielsweise ein Mikrofon 8 vorgesehen ist. Seine nachgesprochenen Worte/Sätze werden aufgenommen und seitens der Software bzw. dem Sprachbeurteilungssystem verarbeitet. Zu diesem Zweck ist eine geeignete Vergleichsstufe und eine Spracherkennungsstufe vorgesehen, die die nachgesprochenen Worte/Sätze analysiert und bewertet.

Ferner ist Software und ein System zur Erfassung kognitiver Fähigkeiten vorgesehen. Hierbei können dem Patienten am Monitor 2 Fragen gestellt werden, die er zu beantworten hat. Zur Eingabe der Antworten kann beispielsweise eine Tastatur 9 verwendet werden, oder aber auch die Bedienmaus 7.

Schließlich ist geeignete Software und ein System zur Erfassung allgemeiner Anamnesedaten wie beispielsweise das momentane Wohlbefinden, etwaige konkrete Schwierigkeiten oder Probleme gleich welcher Art, etc. erfasst werden können. Hierzu sind softwareseitig geeignete Fragenbögen oder wissensbasierte Dialogsysteme vorgesehen, mittels welchem monitorseitig entsprechende Fragen ausgegeben werden können, die der Patient wiederum beispielsweise unter Verwendung der Bedienmaus 7 oder der Tastatur 9 oder aber auch des Mikrofons 8 beantworten muss.

Sämtliche rechnerseitig über die entsprechenden Eingabemittel aufgenommenen Informationen werden software- oder systemseitig vorverarbeitet und an ein rechnerseitig installiertes Expertensystem 10 gegeben. Bei diesem handelt es sich beispielsweise um ein lernendes neuronales Netz. Mittels des Expertensystems wird basierend auf sämtlichen ihm zugetragenen Informationen eine Maßzahl oder Aussage 11 ermittelt, die ein quantifizierendes Maß für den Ist-Zustand des Patienten ist. Ferner ist das Expertensystem zur Ermittlung einer Bewertung 12 betreffend die Dosierung bzw. zur Erarbeitung eines Dosierungsvorschlags ausgebildet. Anhand des Expertensystems wird dabei ermittelt, ob sich der Zustand des Patienten verbessert oder verschlechtert hat oder ob er stabil geblieben ist, wobei nach Erfassung dieses Ergebnisses das Expertensystem bewertet, ob die Dosierung richtig, zu niedrig oder zu hoch ist. Gleichzeitig kann ein Dosierungsvorschlag gemacht werden. Schließlich ist das Expertensystem zur Ermittlung einer Aussage 13 für mögliche Nebenwirkungen einer neuen oder gegebenenfalls der gleichen Dosierung ausgebildet.

Sämtliche in den Schritten 11, 12 und 13 vom Expertensystem ermittelter Maßzahlen, Aussagen und Bewertungen werden einerseits dem Patienten am Ausgabemedium 2 visualisiert, so dass dieser nach Beendigung der Tests bzw. der Informationsaufnahme sofort Kenntnis über das expertensystemseitige Bewertungsergebnis erhält. Ferner wird zumindest die Maßzahl/Aussage 11, gegebenenfalls auch sämtliche anderen Bewertungen/Aussagen in einem rechnerseitigen Speichermedium 14 abgespeichert. Anhand der über die Zeit dort abgelegten Maßzahlen/Aussagen etc. kann eine Verlaufskontrolle abgefragt werden, anhand welcher der zeitliche Verlauf und Trend der Therapie erkannt werden kann. Auch kann die Dosierungsbewertung basierend auf einer Analyse des zeitlichen Verlaufs/Trends der Maßzahlen erfolgen, wobei das Expertensystem zur entsprechenden Analyse der gespeicherten früheren Maßzahlen/Aussagen ausgebildet ist.

Schließlich können die seitens des Expertensystems 10 ermittelten Maßzahlen/Aussagen/Bewertungen über ein rechnerseitig vorgesehenes Übertragungsmittel 15, das an einer nicht näher gezeigte Telekommunikationsleitung angeschlossen ist, an eine externe Rechnereinrichtung 16, die beim behandelnden Arzt steht, übertragen werden (Pfeil 17). Auf diese Weise erhält der Arzt Kenntnis über das Kontroll- bzw. Bewertungsergebnis. In Ansehung dieser Informationen kann der Arzt dann, wie durch den Pfeil 18 angedeutet ist, auch eine Antwort über das Telekommunikationsnetz an den Patienten schicken, dem die Antwort am Ausgabemedium 2 visualisiert wird.

Anhand eines konkreten Fallbeispiels soll die Funktionsweise des Systems näher erläutert werden:

Ein Patient leidet an einer Form der Epilepsie, bei der mit Medikamenten eine weitgehende Unterdrückung der epileptischen Anfälle möglich ist. Als negative Begleiterscheinung der Anfallsunterdrückung führen die eingenommenen Medikamente jedoch gleichzeitig zu einer Verlangsamung der Reaktionsfähigkeit des Patienten und zu einer Verlangsamung der Artikulationsfähigkeit, d.h., in der Unterhaltung klingt die Sprache des Patienten etwas schleppend und langsam, es treten häufig Satzpausen auf. Ziel der Optimierung der Medikamenteneinstellung muss sein, bei ausreichender Anfallsunterdrückung die neurologischen Defizite so gering wie möglich zu halten. Während der Patient typischerweise eine Zunahme der Anfallshäufigkeit selbst sofort bemerkt und dann entweder von sich aus die Dosierung des Medikaments erhöht oder den Arzt aufsucht, sind für ihn subjektiv die Beeinträchtigungen der genannten Fähigkeiten deutlich schlechter beurteilbar, und er wird vor allem eine schleichende Verschlechterung häufig nicht bemerken.

Mit Hilfe des erfindungsgemäßen Systems kann der Patient zuhause, typischerweise etwa jeden zweiten Tag oder auch nur einmal in der Woche, am Computer die Testprogramme ausführen lassen. Einerseits wird beispielsweise seine Reaktionsschnelligkeit im oben beschriebenen Fall mit Hilfe von computerspielartigen Tests erfasst und quantifiziert. Andererseits misst ein Sprachebeurteilungssystem, in dem Texte vorgelesen, nachgesprochen, auf Fragen des Computers geantwortet wird etc., mit Hilfe von Spracherkennungsalgorithmen die Artikulationsfähigkeit. Ferner können bei dem Epileptiker im Rahmen eines interaktiven Dialogs mit dem Computer allgemeine Anamnesedaten über "intelligente Fragenbögen" aufgenommen werden, wie beispielsweise "Haben Sie Schlafstörungen?", "Haben Sie Kopfweh?", "Besteht Übelkeit?", "Fühlen Sie sich gut oder schlecht?" etc. aufgenommen werden. Die Auswertung der Tests und der Allgemeinanamnese erlauben unter Verwendung des Expertensystems eine Aussage, ob die Beeinträchtigung durch Medikamentennebenwirkungen im Trend stabil ist, oder besser oder schlechter wird. Auch können zusätzlich Fragen zur Häufigkeit und Intensität epileptischer Anfälle im Testzeitraum gestellt werden. Das Expertensystem kann nun entsprechende Bewertungen/Aussagen ableiten, wie beispielsweise "Zustand o.k.", "Nächster Test empfohlen in x-Tagen", oder "Ihre Reaktionsfähigkeit hat messbar nachgelassen, erniedrigen Sie Dosis des Medikaments x auf y und wiederholen Sie den Test in n-Tagen".

## Patentansprüche

1. Verfahren zur Selbstkontrolle des Ist-Zustands eines an einer neurologischen Erkrankung leidenden, medikamentös therapierten Patienten, bei welchem unter Verwendung einer patientenseitig angeordneten Rechnereinrichtung Informationen betreffend die motorischen und/oder verbalen und/oder kognitiven Fähigkeiten des Patienten interaktiv erfasst werden und basierend auf diesen Informationen mittels eines Expertensystems wenigstens eine den Zustand beschreibende Maßzahl oder eine Aussage ermittelt und an den Patienten mittels eines rechnerseitigen Ausgabemediums ausgegeben wird.

2. Verfahren nach Anspruch 1, bei welchem der Patient zur Erfassung der die Motorik betreffenden Informationen an der Rechnereinrichtung softwaregesteuerte Motoriktestübungen durchführt, wobei die Motoriktestübungen zur Erfassung der negativen und/oder der positiven Auswirkungen einer Medikamentengabe auf den Patientenzustand dienen und anhand welcher ein die negativen und/oder positiven Auswirkungen quantifizierender Informationswert ermittelt wird, der vom Expertensystem weiterverarbeitet wird.

3. Verfahren nach Anspruch 1 oder 2, bei welchem zur Erfassung der die verbalen Fähigkeiten des Patienten betreffenden Informationen ein rechnerseitig hinterlegtes Sprachbeurteilungssystem verwendet wird, das Spracherkennungsalgorithmen und/oder eine phonetische Datenbank enthält, mittels welchem ein die negativen und/oder positiven Auswirkungen einer Medikamentengabe quantifizierender Informationswert der Artikulationsfähigkeit ermittelt wird, der vom Expertensystem weiterverarbeitbar ist.

4. Verfahren nach einem der vorangehenden Ansprüche, bei welchem zur Erfassung der kognitiven Fähigkeiten des Patienten softwaregesteuert im Frage-Antwort-Verfahren vorgegebene Fragen optisch oder akustisch ausgegeben werden, die der Patient verbal oder manuell rechnerseitig beantworten muss, wobei die Antworten beurteilt und darauf basierend ein die negativen und/oder positiven Auswirkungen quantifizierender Informationswert der kognitiven ermittelt wird, der vom Expertensystem weiterverarbeitet wird.

5. Verfahren nach einem der vorangehenden Ansprüche, bei welchem unter Verwendung eines Erfassungssystems rechnerseitig einzugebende oder verbal erfassbare Informationen betreffend das subjektive Befinden des Patienten erfasst werden, die seitens des Expertensystems weiterverarbeitet werden.

6. Verfahren nach einem der vorangehenden Ansprüche, bei welchem die Maßzahl oder die Aussage, gegebenenfalls auch die Informationswerte rechnerseitig zeitbezogen gespeichert werden.

7. Verfahren nach einem der vorangehenden Ansprüche, bei welchem das Expertensystem anhand der gegebenen Maßzahl oder Aussage oder der Informationen bzw. des ermittelten Informationswerts eine Bewertung betreffend die Medikamentendosierung vornimmt und ausgibt.

8. Verfahren nach Anspruch 6 und 7, bei welchem die Bewertung basierend auf einer Analyse der zeitlichen Änderung bzw. des zeitlichen Verlaufs der Maßzahl oder der Aussge ermittelt wird.

9. Verfahren nach einem der Ansprüche 6 bis 8, bei welchem gespeicherte Maßzahlen über die Zeit als Verlaufsdiagramm ausgegeben werden können.

10. Verfahren nach einem der Ansprüche 7 bis 9, bei welchem die Bewertungsergebnisse rechnerseitig gespeichert werden.

11. Verfahren nach einem der vorangehenden Ansprüche, bei welchem die Maßzahl oder die Aussage, gegebenenfalls auch die Bewertung betreffend die Medikamentendosierung und gegebenenfalls die Informationen selbst über eine Kommunikationsverbindung an einen Arzt übertragen werden.

12. Verfahren nach Anspruch 11, bei welchem der Arzt über die Kommunikationsverbindung in Ansehung der übertragenen Maßzahlen oder Aussagen oder Informationen eine Beurteilung des Patientenzustands vornimmt und eine Therapieanweisung an den Patienten übermittelt, die dem Patienten am Ausgabemedium angezeigt werden.

13. System zur Durchführung des Verfahrens nach einem der Ansprüche 1 bis 12, umfassend eine patientenseitig angeordnete Rechnereinrichtung (1), in welcher ein oder mehrere Softwareprogramme (3, 4, 5, 6) abgelegt sind, mittels denen dem Patienten an einem oder mehreren Ausgabemedien interaktive Testübungen betreffend die motorischen und/oder verbalen und/oder kognitiven Fähigkeiten des Patienten optisch und/oder akustisch ausgebbar sind, wobei die Rechnereinrichtung (1) zur Erfassung der Antwortinformationen des Patienten ausgebildet ist, und wobei rechnerseitig ein Expertensystem (10) vorgesehen ist, das zur Ermittlung wenigstens einer an einem Ausgabemedium (2) ausgebbaren, den Zustand des Patienten beschreibenden Maßzahl oder einer Aussage basierend auf den Antwortinformationen ausgebildet ist.

14. System nach Anspruch 13, bei welchem unter Steuerung über wenigstens ein rechnerseitiges Softwareprogramm (3) dem Patienten Motoriktestübungen an einem Ausgabemedium (2) visualisierbar und Informationen betreffend das Antwortverhalten des Patienten rechnerseitig erfassbar ist, wobei anhand der Informationen ein die negativen und/oder positiven Auswirkungen quantifizierender Informationswert ermittelbar ist, der vom Expertensystem (10) weiterverarbeitbar ist.

15. System nach Anspruch 13 oder 14, bei welchem eine Sprachaufnahmeeinrichtung (8) und ein rechnerseitig vorgesehenes Sprachbeurteilungssystem (4) zur Erfassung der die verbalen Fähigkeiten des Patienten betreffenden Informationen vorgesehen sind, welches Spracherkennungsalgorithmen und/oder eine phonetische Datenbank enthält, und mittels welchem ein quantifizierbarer Informationswert der Artikulationsfähigkeit ermittelbar ist, der vom Expertensystem (10) weiterverarbeitbar ist.

16. System nach einem der Ansprüche 13 bis 15, bei welchem unter Steuerung über wenigstens ein rechnerseitig abgelegtes Softwareprogramm (5) vorgegebene Fragen optisch oder akustisch ausgegeben werden, wobei die verbalen oder rechnerseitig manuell eingebbaren Antworten des Patienten erfassbar sind und darauf basierend ein die negativen und/oder positiven Auswirkungen quantifizierender Informationswert der kognitiven Fähigkeiten ermittelbar ist, der vom Expertensystem (10) weiterverarbeitbar ist.

17. System nach einem der Ansprüche 13 bis 16, bei welchem ein rechnerseitiges Erfassungssystem (6) vorgesehen ist, mittels welchem in einem Frage-Antwort-Verfahren rechnerseitig einzugebende oder verbal erfassbare Informationen betreffend das subjektive Befinden des Patienten aufnehmbar sind, welche vom Expertensystem weiterverarbeitbar sind.

18. System nach einem der Ansprüche 13 bis 17, bei welchem bei welchem das Expertensystem (10) zur Ermittlung einer Bewertung betreffend die Medikamentendosierung anhand der erfassten Antwortinformationen bzw. der ermittelten Informationswerte bzw. der ermittelten Maßzahl oder Aussage ausgebildet ist.

19. System nach einem der Ansprüche 13 bis 18, bei welchem rechnerseitig wenigstens ein Speichermittel (14) zum Speichern der Maßzahl oder der Aussage, gegebenenfalls auch der Informationswerte und der Bewertungsergebnisse vorgesehen ist.

20. System nach Anspruch 18 und 19, bei welchem das Expertensystem (10) zur Analyse des zeitlichen Verlaufs bzw. der zeitlichen Änderung der gespeicherten Maßzahlen oder Aussagen und zur Ermittlung der Bewertung in Abhängigkeit des Analyseergebnisses ausgebildet ist.

21. System nach Anspruch 19 oder 20, bei welchem gespeicherte Maßzahlen über die Zeit als Verlaufsdiagramm am Ausgabemedium (2) ausgebbar sind.

22. System nach einem der Ansprüche 13 bis 21, bei welchem Übertragungsmittel (15) zum Übertragen der Maßzahl oder der Aussage, gegebenenfalls auch der Bewertung betreffend die Medikamentendosierung und gegebenenfalls der Informationen selbst über eine Kommunikationsverbindung an eine externe Rechnereinrichtung (16) vorgesehen sind.
